# FASCICULE DE BREVET EUROPEEN

(11) **EP 1 841 479 B1**
(45) Date de publication et mention de la délivrance du brevet: **31.08.2016**
(21) Numéro de dépôt: 05824667.9
(22) Date de dépôt: 13.12.2005
(51) Int. Cl.: A61M 15/00

(54) **DISPOSITIF DE DISTRIBUTION DE PRODUIT FLUIDE**
VORRICHTUNG ZUR ABGABE EINES FLÜSSIGEN PRODUKTS
DEVICE FOR DISPENSING A FLUID PRODUCT

(30) Priorité: 17.12.2004 FR 0453057
(43) Date de publication de la demande: 10.10.2007
(73) Titulaire: Aptar France SAS, 27110 Le Neubourg (FR)
(72) Inventeur: PARDONGE, Jean-Marc, F-76520 Les Authieux sur Port Saint Ouen (FR); HAFFAR, Salim, F-78620 L'etang la Ville (FR)
(74) Mandataire: CAPRI
(86) Numéro de dépôt international: PCT/FR2005/051078
(87) Numéro de publication internationale: WO 2006/064159

(56) Documents cités:
- EP-A- 1 475 116
- WO-A-00/16836
- WO-A-92/17231
- WO-A-02/092152
- WO-A2-2004/041334
- FR-A1- 2 842 905
- US-A- 5 664 557
- US-A1- 2003 183 226
- US-A1- 2004 089 292
- US-B1- 6 305 371

## Description

La présente invention concerne un dispositif de distribution de produit fluide.

Les domaines d'application privilégiés d'un tel dispositif sont notamment mais pas exclusivement la pharmaceutique, la cosmétique et la parfumerie.

Les dispositifs de distribution de l'art antérieur comprennent généralement un réservoir de produit fluide sur lequel est monté un organe de distribution tel qu'une pompe ou une valve. Cet organe comprend généralement une chambre de dosage dans laquelle une tige d'actionnement ou soupape est montée coulissante. En particulier dans le cas de dispositifs aérosols, dans lesquels le produit est expulsé au moyen d'un gaz propulseur à travers une valve doseuse, le réservoir est généralement monté coulissant dans le corps pour actionner la soupape de valve. Le raccordement du corps avec la soupape monté sur le réservoir définit une position d'utilisation du dispositif dans laquelle ladite soupape coopère avec le corps. Dans cette position d'utilisation, la soupape est alors déplaçable entre une position de repos et une position de distribution suite à un actionnement par l'utilisateur. Généralement, il est nécessaire au bout de plusieurs actionnements de la soupape de nettoyer l'interface entre la soupape et le corps. En effet, des reliquats de produit fluide distribués peuvent se déposer sur les parois du manchon de raccordement coopérant avec la soupape. Or, ces reliquats sont susceptibles de polluer le produit à distribuer restant dans le réservoir voire même d'entraver la sortie de produit fluide. Pour ce nettoyage, l'utilisateur doit alors désengager la soupape du manchon de raccordement et sortir le réservoir hors du corps. Ainsi, ce désengagement donne lieu à une position de retrait du dispositif dans lequel la soupape ne coopère pas avec le corps. Le corps ainsi que le réservoir sont alors susceptibles d'être égarés ou alors, suite à une opération de nettoyage, il existe des risques d'interchanger des corps et des réservoirs appartenant à des utilisateurs différents. Cette situation peut notamment se produire dans des services de soins hospitaliers au niveau desquels les dispositifs de distribution sont nettoyés en série. Ainsi, si deux réservoirs sont interchangés, il existe des risques éventuels de problèmes hygiéniques et notamment de contaminations microbiologiques pour l'utilisateur notamment lorsque le dispositif de distribution est un dispositif de distribution par voie buccale. D'autre part, si des réservoirs ne portant pas d'informations sur l'identité du produit qu'ils contiennent sont intervertis, alors l'utilisateur dans le cas de produit pharmaceutique, peut absorber ou inhaler un produit contre-indiqué ayant des effets allergisants ou susceptibles d'engendrer des incompatibilités médicamenteuses. Le fait d'interchanger deux réservoirs après un nettoyage est particulièrement dangereux lorsque le dispositif est pourvu d'un compteur ou indicateur de doses, en particulier lorsque ce compteur est solidaire du corps. En effet, une erreur de réservoir pourrait avoir des conséquences dramatiques si l'utilisateur se retrouve avec un compteur affichant un nombre de doses distribué inférieur à celui restant en réalité dans le réservoir. En effet, l'utilisateur risque alors de se retrouver avec un réservoir vide alors que le compteur lui indique qu'il reste des doses à distribuer. Le risque pour la santé est alors très important.

Les documents WO 92 /17231 et US 2003/183226 divulguent des systèmes électroniques très complexes et coûteux permettant d'associer un réservoir à un inhalateur.

D'autres dispositifs de distribution sont connus de FR 2 842 905 et WO 00/16836. Le document WO 2004/041334 divulgue un dispositif de distribution comportant les caractéristiques du préambule de la revendication 1.

La présente invention a pour but de fournir un dispositif de distribution qui ne reproduit pas les inconvénients susmentionnés.

En particulier, la présente invention a pour but de fournir un dispositif de distribution de produit fluide qui évite le risque d'interchanger par erreur le réservoir, notamment après nettoyage du dispositif.

Plus particulièrement, la présente invention a pour but de fournir un dispositif de distribution qui soit simple et peu coûteux à fabriquer et à assembler.

La présente invention a donc pour objet un dispositif de distribution de produit fluide selon la revendication 1.

Selon l'invention les moyens d'identification du réservoir sont identiques aux moyens d'identification du corps. Ainsi, un seul réservoir et un seul corps comportent le même marquage, de sorte que l'utilisateur évitera d'interchanger accidentellement des réservoirs après nettoyage du corps, au moment où le réservoir est remis en place dans le corps.

Avantageusement, lesdits moyens d'identification sont visuels et/ou tactiles.

Avantageusement, lesdits moyens d'identification comportent des marquages, notamment réalisés par gravure et/ou impression.

Selon l'invention un seul réservoir comporte des moyens d'identification associés aux moyens d'identification d'un corps spécifique.

Selon l'invention ledit corps est muni d'un dispositif de comptage ou d'indication des doses de produit fluide distribuées ou restant à distribuer à partir du réservoir.

Selon une caractéristique intéressante de l'invention, ledit dispositif d'indication ou de comptage est actionné par le déplacement du réservoir entre ses positions de repos et de distribution.

Selon l'invention lesdits moyens d'identification sont dépourvus de moyens électroniques.

D'autres caractéristiques et avantages de la présente invention apparaîtront plus clairement au cours de la description détaillée suivante d'un mode de réalisation de l'invention, faite en référence aux dessins joints, donnés à titre d'exemples non limitatifs, et sur lesquels :

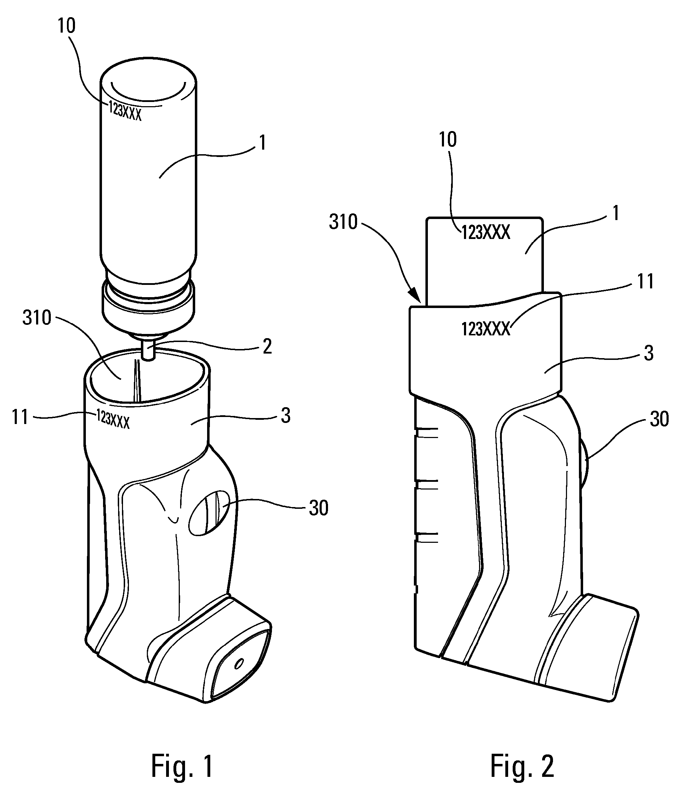

- la figure 1 est une vue schématique en perspective d'un dispositif de distribution selon l'invention avec le réservoir séparé du corps, et
- la figure 2 est une vue schématique de côté du dispositif de distribution de la figure 1 avec le réservoir inséré dans le corps.

Le dispositif de distribution, selon la présente invention comporte un réservoir 1, un organe de distribution 2, et un corps 3, le réservoir 1 étant assemblé de manière amovible dans ledit corps 3.

Le réservoir 1 peut présenter toute forme appropriée. Ce réservoir peut en particulier comporter un fût généralement cylindrique contenant du produit fluide à pression atmosphérique ou du produit fluide pressurisé.

L'organe de distribution 2 peut être une pompe ou une valve, de préférence une valve doseuse, dont le fonctionnement ne sera pas expliqué plus en détail. Dans le cas d'une valve, généralement utilisée avec des dispositifs du type aérosol, on prévoit une soupape déplaçable dans une chambre de dosage pour expulser la dose du produit au moyen d'un gaz propulseur. Le réservoir 1 est monté coulissant axialement dans ledit corps. Ce déplacement axial provoque le déplacement de la soupape et donc l'actionnement de la valve permettant ainsi le vidage de ladite chambre de dosage.

Le corps 3 est selon le mode de réalisation représenté sur les différentes figures un corps typique pour inhalateur. Ce corps peut comporter une coque sensiblement cylindrique comprenant une extrémité supérieure définissant une ouverture 310 recevant le réservoir 1. Il est à noter que le corps tel que représenté n'est qu'un exemple de réalisation. Des corps de toutes autres configurations pourraient très bien être envisagés tels que ceux conventionnellement utilisés pour des applications nasales, otologiques ou encore dans le domaine de la parfumerie ou de la cosmétique.

Le réservoir 1 est introduit dans le corps 3 par l'ouverture 310. Ensuite, le réservoir 1 est assemblé dans le corps 3 par emmanchage de la soupape dans un manchon de raccordement (non représenté). Dans le cas présent, l'actionnement du dispositif de distribution représenté peut se faire de façon classique en appuyant sur le fond du réservoir 1. En utilisation, la valve coopère avec le corps 3 entre une position de repos et une position de distribution.

Selon l'invention, des moyens d'identification 10, 11 sont respectivement prévus sur le réservoir 1 et le corps 3 pour associer toujours le même réservoir au même corps, par exemple après un nettoyage ayant impliqué un retrait du réservoir hors du corps. Ces moyens d'identification sont identiques sur le réservoir et le corps et uniques, c'est-à-dire qu'un même marquage n'apparaît que sur un seul réservoir associé à un seul corps. Par exemple, on peut imaginer un numéro, un symbole, ou tout autre moyen approprié. Ces moyens d'identification peuvent être visuels ou tactiles, par exemple en braille pour les personnes non-voyantes. Avantageusement, comme représenté sur les dessins, les moyens d'identification sont disposés à proximité les uns des autres lorsque le réservoir est en position assemblée, pour alerter visuellement l'utilisateur en cas d'erreur.

Les moyens d'identification peuvent être réalisés par exemple par gravage ou impression. En variante, on peut envisager l'utilisation d'étiquettes appropriées. D'autres variantes sont aussi envisageables, tel que la sérigraphie. Avantageusement, ces moyens d'identifications sont apposés sur le dispositif au moment de l'assemblage en usine du réservoir dans le corps.

Un avantage particulier de l'invention est de pouvoir associer un réservoir avec un corps de manière très simple et peu coûteuse. En particulier, les moyens d'identification de l'invention sont dépourvus de moyens électroniques ou similaires.

Selon l'invention le corps 3 est muni d'un dispositif 30 de comptage ou d'indication des doses de produit fluide distribuées ou restant à distribuer à partir du réservoir 1. Comme représenté, ce compteur peut faire partie dudit corps 3. Ce dispositif de comptage ou d'indication peut être actionné par le déplacement du réservoir entre ses positions de repos et de distribution. Grâce à la présence des moyens d'identification 10, 11, l'utilisateur ou le préposé au nettoyage ne risque pas d'interchanger accidentellement les réservoirs après un nettoyage. Par conséquent, le même réservoir 1 sera toujours associé à son corps respectif 3 et le nombre de doses indiqué au niveau du compteur 30 du corps correspondra forcément au bon nombre de doses distribuées ou restant à distribuer dans le réservoir 1.

Bien que la présente invention ait été décrite en référence à un mode de réalisation particulier de celle-ci, il est clair qu'elle n'est pas limitée par ce mode de réalisation. Au contraire, un homme du métier peut y apporter toute modification utile sans sortir du cadre de la présente invention tel que défini par les revendications annexées.

## Revendications

1. Dispositif de distribution de produit fluide comprenant :
- un réservoir de produit fluide (1) à distribuer,
- un organe de distribution (2), tel qu'une pompe ou une valve monté sur ledit réservoir (1), et
- un corps (3) apte à recevoir ledit réservoir (1), ledit corps (3) étant pourvu d'un orifice de distribution et d'une ouverture (310) par laquelle ledit réservoir (1) peut être introduit dans le corps (3), ledit réservoir (1) étant déplaçable dans ledit corps (3) entre une position de repos et une position de distribution, ledit corps (3) étant muni d'un dispositif de comptage ou d'indication des doses (30) de produit fluide distribuées ou restant à distribuer à partir du réservoir (1), ledit dispositif d'indication ou de comptage (30) étant actionné par le déplacement du réservoir (1) dans le corps (3) entre ses positions de repos et de distribution, ledit réservoir (1) et ledit corps (3) comportant respectivement des moyens d'identification (10, 11) qui permettent d'associer ledit réservoir (1) audit corps (3), les moyens d'identification (10) du réservoir (1) étant identiques aux moyens d'identification (11) du corps (3) lesdits moyens d'identification (10, 11) étant dépourvus de moyens électroniques, **caractérisé en ce que** ledit réservoir (1) est amovible dudit corps (3) et **en ce que** les moyens d'identification (10, 11) sur le réservoir (1) et le corps (3) sont uniques, c'est à dire qu'un même moyen d'identification n'apparaît que sur un seul réservoir associé à un seul corps.

2. Dispositif selon la revendication 1, dans lequel lesdits moyens d'identification (10, 11) sont visuels et/ou tactiles.

3. Dispositif selon l'une quelconque des revendications précédentes, dans lequel lesdits moyens d'identification (10, 11) comportent des marquages, notamment réalisés par gravure et/ou impression.

## Patentansprüche

1. Ausgabevorrichtung für ein fluides Produkt, aufweisend:
- einen Behälter für auszugebendes fluides Produkt (1),
- eine Ausgabeeinrichtung (2), wie eine Pumpe oder ein Ventil, die auf dem Behälter (1) montiert ist, und
- einen Körper (3), der dazu geeignet ist, den Behälter (1) aufzunehmen, wobei der Körper (3) mit einer Ausgabeöffnung und einer Öffnung (310) versehen ist, durch die der Behälter (1) in den Körper (3) eingeführt werden kann, wobei der Behälter (1) in dem Körper (3) zwischen einer Ruheposition und einer Ausgabeposition verschiebbar ist,
wobei der Körper (3) mit einer Zähl- oder Anzeigevorrichtung für Dosen (30) von fluidem Produkt, die aus dem Behälter (1) ausgegeben wurden oder noch auszugeben sind, versehen ist, wobei die Anzeige- oder Zählvorrichtung (30) durch die Verschiebung des Behälters (1) in dem Körper (3) zwischen seiner Ruheposition und seiner Ausgabeposition betätigt wird, wobei der Behälter (1) und der Körper (3) jeweils Identifizierungsmittel (10, 11) aufweisen, durch die der Behälter (1) dem Körper (3) zugeordnet werden kann, wobei die Identifizierungsmittel (10) des Behälters (1) mit den Identifizierungsmitteln (11) des Körpers (3) identisch sind, wobei die Identifizierungsmittel (10, 11) frei von elektronischen Mitteln sind, **dadurch gekennzeichnet, dass** der Behälter (1) vom Körper (3) abnehmbar ist und dass die Identifizierungsmittel (10, 11) auf dem Behälter (1) und dem Körper (3) einmalig sind, das heißt, dass dasselbe Identifizierungsmittel nur auf einem einzigen Behälter erscheint, der einem einzigen Körper zugeordnet ist.

2. Vorrichtung nach Anspruch 1, wobei die Identifizierungsmittel (10, 11) sichtbar und/oder tastbar sind.

3. Vorrichtung nach einem der vorhergehenden Ansprüche, wobei die Identifizierungsmittel (10, 11) Markierungen aufweisen, die insbesondere durch Gravur und/oder Druck angebracht sind.

## Claims

1. A fluid dispenser device comprising:
• a reservoir (1) of fluid to be dispensed;
• a dispenser member (2), such as a pump or a valve, mounted on said reservoir (1); and
• a body (3) that is suitable for receiving said reservoir (1), said body (3) being provided with a dispenser orifice and an opening (310) through which said reservoir (1) can be inserted into the body (3), said reservoir (1) being displaceable in said body (3) between a rest position and a dispensing position, said body (3) being provided with a counter or indicator device (30) for counting or indicating the doses of fluid that have been dispensed or that remain to be dispensed from the reservoir (1), said counter or indicator device (30) being actuated by displacing the reservoir (1) in the body (3) between its rest and dispensing positions, said reservoir (1) and said body (3) including respective ID means (10, 11) that make it possible to associate said reservoir (1) with said body (3), the ID means (10) of the reservoir (1) being identical to the ID means (11) of the body (3), said ID means (10, 11) having no electronic means, the device being **characterized in that** said reservoir (1) is removable from said body (3) and **in that** the ID means (10, 11) on the reservoir (1) and the body (3) are unique, that is to say that the same ID means only appears on one single reservoir associated with one single body.

2. A device according to claim 1, in which said ID means (10, 11) are visual and/or tactile.

3. A device according to any preceding claim, in which said ID means (10, 11) include markings, in particular made by etching and/or printing.
